# EUROPÄISCHE PATENTSCHRIFT

(11) **EP 0 542 119 B1**
(45) Veröffentlichungstag und Bekanntmachung des Hinweises auf die Patenterteilung: **30.07.1997**
(21) Anmeldenummer: 92118895.9
(22) Anmeldetag: 04.11.1992
(51) Int. Cl.: A61F 2/52

(54) **Verfahren zur Herstellung einer Brustprothese**
Process of manufacturing a mammary prosthesis
Procédé de fabrication d'une prothèse mammaire

(30) Priorität: 14.11.1991 DE 9114201 U; 21.11.1991 DE 9114512 U
(43) Veröffentlichungstag der Anmeldung: 19.05.1993
(73) Patentinhaber: AMOENA Medizin-Orthopädie-Technik GmbH, D-83064 Raubling (DE)
(72) Erfinder: Wild, Helmut Franz, W-8201 Rohrdorf (DE)
(74) Vertreter: Gossel, Hans K., Dipl.-Ing.

(56) Entgegenhaltungen:
- EP-A- 0 392 960
- EP-A- 0 433 636
- DE-A- 2 742 394
- DE-A- 2 802 375
- DE-U- 9 107 507
- GB-A- 2 202 745

## Beschreibung

Die Erfindung betrifft ein Verfahren zur Herstellung einer Brustprothese.

Ein besonderes Problem bei Brustprothesen besteht darin, diese gut und möglichst unverrutschbar an der Brust der Trägerin zu befestigen.

Bei einer aus dem DE-GM 90 10 426 bekannten Brustprothese dieser Art erfolgt die Befestigung dadurch, daß die Prothesenkörper in die Körbchen eines Büstenhalters eingelegt werden, die zusätzlich zur Halterung der Prothesenkörper mit Einlegetaschen versehen sein können.

Eine besonders günstige Halterung einer Brustprothese ist dadurch erreichbar, wenn diese unmittelbar auf der Haut, bzw. dem Narbenbereich, der Trägerin befestigt wird. Eine aus EP-A-392 960 bekannte Brustprothese der eingangs angegebenen Art ist innerhalb eines umlaufenden lippenförmigen Randes auf ihrer Rückseite mit einer umlaufenden, durch einen Absatz gebildeten Schulter versehen, auf der Haftstreifen oder Haftstücke befestigt sind, die mit den Haftbereichen von auf den Körper der Frau durch hautfreundliche Klebemittel befestigte Streifen in der Weise zusammenwirken, daß die Prothese mit den auf der Haut klebenden Haltestreifen verbunden und von diesen wieder gelöst werden kann. Als bevorzugtes Befestigungsmittel ist dabei eine Klettverbindung vorgesehen. Diese Art der Befestigung ist relativ umständlich, weil die Frau zum Anlegen der Prothese zunächst auf ihre Haut einen Haftstreifen kleben muß, der auf seiner Außenseite als Teil einer Klettverbindung ausgebildet ist oder einen Verbindungsbereich aufweist, der haftend mit der nachträglich aufgesetzten Prothese verbunden werden kann.

Aus der DE-A-2 802 375 ist eine Brustprothese aus einer porigen Silikon-Kautschuk-Masse bekannt, auf deren Rückseite Kuststoff-Folien-Streifen aus Polyurethan mittels einer kalt vulkanisierenden Paste ans Prothesenmaterial anvulkanisiert wird. Diese Kunststoff-Folien-Streifen bilden Anbringungsstellen für Kuststoff-Haftstreifen, die beiderseits mit einem hautverträglichen Kleber beschichtet sind.

Aufgabe der Erfindung ist es daher, Verfahren zur Herstellung einer Brustprothese der eingangs angegebenen Art zu schaffen, die sich in einfacher Weise lösbar unmittelbar auf der Haut einer Trägerin befestigen läßt.

Erfindungsgemäß wird diese Aufgabe durch die Ansprüche 1 und 2 gelöst.

Die dauernd klebrig bleibende Schicht aus einer klebrig eingestellten additionsvernetzenden Zwei-Komponenten-Silikon-Kautschuk-Masse ist auf eine Tragfolie aufgebracht, die mit der die Prothese einhüllenden Folie verklebt oder verschweißt wird. Die dauernd klebrig bleibende Schicht aus einer klebrig eingestellten additionsvernetzenden Zwei-Komponenten-Silikon-Kautschuk-Masse wird zusammen mit der Tragfolie in eine Form eingebracht und in dieser ausgehärtet.

Die die dauernd klebrig bleibende Schicht tragende Tragfolie ist gemäß Anspruch 2 mit der Prothesenhülle durch einen Schmelzkleber verklebt. Eine derartige Verklebung erfolgt bei der Herstellung der Prothese dadurch, daß die Tragfolie auf die entsprechende Stelle der Prothese aufgebracht wird und dann während des Aushärtungsprozesses mit der Prothesenhülle verklebt.

## Patentansprüche

1. Verfahren zur Herstellung einer Brustprothese, wobei
a) ein schalenförmiger Körper aus einer weich-elastisch eingestellten additionsvernetzenden Zwei-Komponenten-Silikon-Kautschuk-Masse, der in Kunststoffolien eingeschweißt ist, die dessen Außenseite und dessen Innenseite abdecken, hergestellt wird,
b) eine klebrig eingestellte additionsvernetzende Zwei-Komponenten-Silikon-Kautschuk-Masse zur Bildung einer dauernd klebrig bleibenden Schicht mit einer Tragfolie in eine Form eingebracht wird und in dieser ausgehärtet wird und
c) die Tragfolie mit der die Prothese einhüllenden Folie auf ihrer Rückseite in ihrem Randbereich verklebt oder verschweißt wird.

2. Verfahren zur Herstellung einer Brustprothese, wobei
a) eine klebrig eingestellte additionsvernetzende Zwei-Komponenten-Silikon-Kautschuk-Masse zur Bildung einer dauernd klebrig bleibenden Schicht mit einer Tragfolie in eine Form eingebracht wird und in dieser ausgehärtet wird und auf der anderen Seite der Tragfolie ein Schmelzkleber aufgebracht wird und
b) ein schalenförmiger Körper aus einer weich-elastisch eingestellten additionsvernetzenden Zwei-Komponenten-Silikon-Kautschuk-Masse, der in Kunststoffolien eingeschweißt ist, die dessen Außenseite und dessen Innenseite abdecken, hergestellt wird und in einem Aushärtungsprozeß ausgehärtet wird und während dieses Aushärtungsprozesses auf der Rückseite der Brustprothese in ihrem Randbereich die Tragfolie durch den Schmelzkleber verklebt wird.

## Claims

1. Process for manufacturing a mammary prosthesis, comprising the following steps
a) a shell-shaped body, which is sealed in films of plastic which cover its outer side and its inner side, is produced from an addition-crosslinking two-component silicone rubber composition of a pliable formulation,
b) for forming a layer which permanently remains tacky, an addition-crosslinking two-component silicone rubber composition of a tacky formulation is introduced with a supporting film into a mould and is cured in the latter, and
c) the supporting film is adhesively bonded or welded to the film enveloping the prosthesis, in the edge region of the rear side of the said film.

2. Process for manufacturing a mammary prosthesis, comprising the following steps
a) for forming a layer which permanently remains tacky, an addition-crosslinking two-component silicone rubber composition of a tacky formulation is introduced with a supporting film into a mould and is cured in the latter, and a hot-melt adhesive is applied to the other side of the supporting film, and
b) a shell-shaped body, which is sealed in films of plastic which cover its outer side and its inner side, is produced from an addition-crosslinking two-component silicone rubber composition of a pliable formulation, and is cured in a curing process and, during this curing process, the supporting film is adhesively bonded by the hot-melt adhesive on the rear side of the mammary prosthesis, in the edge region of the latter.

## Revendications

1. Procédé de fabrication d'une prothèse mammaire, où
a) un corps en forme de coupe en une masse de caoutchouc aux silicones à deux composants prévu pour être mou et élastique, qui est soudé dans des feuilles en matière synthétique qui recouvrent sa face externe et sa face interne, est fabriqué
b) une masse de caoutchouc aux silicones à deux composants prévu pour être collant et se réticulant par addition pour la formation d'une couche collante en permanence est mise en place avec une feuille de support dans un moule et y est durcie
c) la feuille de support avec la feuille enveloppant la prothèse sur sa face arrière est collée ou soudée dans sa zone de bordure.

2. Procédé pour la fabrication d'une prothèse mammaire où
a) une masse de caoutchouc aux silicones à deux composants prévu pour être collant et se réticulant par addition pour la formation d'une couche restant collante en permanence est placée dans un moule avec une feuille de support et y est durcie et sur l'autre face de la feuille de support est appliquée une colle fusible et
b) un corps en forme de coupe en une masse en caoutchouc aux silicones à deux composants prévu pour être mou et élastique et se réticulant par addition, qui est soudé dans les feuilles de matière synthétique qui recouvrent sa face externe et sa face interne, est fabriqué et est durci dans un processus de durcissement et pendant ce processus de durcissement, sur la face arrière de la prothèse mammaire, dans sa zone de bordure, la feuille de support se trouve collée par la colle fusible.
